# EUROPEAN PATENT APPLICATION

(11) **EP 2 494 956 A1**
(43) Date of publication of application: **05.09.2012**
(21) Application number: 10826098.5
(22) Date of filing: 28.10.2010
(51) Int. Cl.: A61K 9/107, A61K 31/337, A61K 47/28, A61P 35/00

(54) **PACLITAXEL/STEROIDAL COMPLEX**

(30) Priority: 29.10.2009 CN 200910236960
(71) Applicant: INSTITUTE OF MATERIA MEDICA, CHINESE ACADEMY OF MEDICAL SCIENCES, Beijing 100050 (CN)
(72) Inventor: LIU, Yuling, Beijing 100050 (CN); Xia, Xuejun, Beijing 100050 (CN); GUO, Ruifang, Beijing 100050 (CN); ZHANG, Pengxiao, Beijing 100050 (CN); ZHOU, Cuiping, Beijing 100050 (CN); WANG, Renyun, Beijing 100050 (CN); JIN, Dujia, Beijing 100050 (CN)
(74) Representative: Hutter, Anton
(86) International application number: PCT/CN2010/078202
(87) International publication number: WO 2011/050735

(57) **Abstract**

A paclitaxel/steroid complex comprising paclitaxel and steroid is disclosed. The molar ratio of paclitaxel to steroid is 1:0.2~4, preferably 1:0.25~2. A process for the preparation thereof and the use thereof in the manufacture of submicron emulsion, dry emulsion, self-microemulsifying system are also disclosed.

## Description

### Field of the Invention

The present invention relates to a kind of paclitaxel steroid complex and its preparation, and the present invention also relates to the use of paclitaxel steroid complex, which belongs to the technical field of pharmaceutical preparations.

### Background of the Invention

Paclitaxel (paditaxel, Taxol) has a significant anti-tumor activity, which is widely used in clinical treatment of ovarian cancer, breast cancer, non-small cell lung cancer, head cancer and neck cancer, etc. Due to its practical insoluble in water (0.006µg/ml), the common injection Taxol® intended for the clinical treatment is prepared by dissolving 30mg of paclitaxel with 5ml of mixed solution containing Cremopher EL (polyoxyethylene castor oil)/ ethanol (50:50, V/V) . However, the solvent containing a large amount of Cremopher EL in formulation releases histamine in vivo, which can cause severe allergic reaction. In order to avoiding such side effects, the desensitization is to be done as followed before the clinical use: 10mg of dexamethasone is given by oral administration 12 hours before the treatment and 10mg of dexamethasone given again by oral administraion 6 hours before the treatment, 20mg of diphenhydramine i.m., 300mg of cimetidine or 50mg of ranitidine i.v. given 30~60 minutes before the treatment. In spite of precaution, 5% to 30% of patients has different degrees of allergic reaction in clinical practice. Moreover, the physical stability issue of paclitaxel solution is to set forth after diluting with Cremopher EL/ethanol, for example, the drug may be precipitated due to lower temperature or long infusion time, which can cause the safety risk.

As for paclitaxel injection issue, pharmacists at home and abroad have carried out excesstive study of new drug delivery system since paclitaxel marketed over 20 years, and related technical solution involves in cyclodextrin inclusion complex, liposome, polymeric micelles and nanoparticles, etc.

Cyclodextrin inclusion complex can improve the solubility of paclitaxel, but the large quantities of cyclodextrin inclusion complex lead to servere renal toxicity and producing precipitation after diluting with water, therefore it failed to the clinical stage so far.

Liposome has lots of shortcoming in terms of low encapsulation efficiency, easy leakage for the long-term storage, and producing precipitation after diluting with water, and industrialized development is struggling. Although overseas has been carried out the research for 20 years, but no product marketed. Paclitaxel liposome for injection (Li Pusu) containing 30mg of medicament per vial, its specification and dose for clinical use comply with the commercially available injection. There is no significant difference in efficacy, and the intermediate preparation process as well as desensitization pretreatment before use has been added, thus can not reflect the obvious advantages of technology.

The domestic and foreign research is extremely active in paclitaxel polymeric micelles, due to its low drug loading and unstable quality after storage, it is to should be frozen to store which limits the development of the industry. In recent years, with the emergence of novel polymer materials, polymeric micelles technology has been developed rapidly, but the new structure of the polymer material into the body, its clinical safety of drug needs to be further investigated.

Paclitaxel nanoparticles combined with albumin for injection (Code No. ABI-007) in American Bioscience Company approved by FDA in 2005 is by far the most influential paclitaxel preparation protected by the PCT patent and its technical solution is prepared as the following steps: using human serum albumin as drug carrier, produce paclitaxel into nanoparticles combined with albumin, sterilize, filter and freeze to drying, obtain paclitaxel injection containning 100mg of paclitaxel and 900mg of albumin per vial. Compared with the common injection, paclitaxel nanoparticles combined with albumin has two advantages, 1) there is no Cremopher EL in the formulation which eliminates the allergic reaction completely and becomes the only paclitaxel preparation for direct use without desensitization treatment in the international; 2) due to toxicity reduction and tolerability improvement, the dose amount administered by patient increases from 135 ~ 175mg/m² to 260mg/m², the crative effect is superior to that of the common paclitaxel injection. Albumin as the carrier with the characteristics of large amounts and expensive cost (the price up to 6200 RMB per vial), as well as the complex of the intermediate procedure, limits the clinical application ofpaclitaxel nanoparticles combined with albumin.

For oil-in-water submicron emulsion, taking advantage of drug lipophilicity, dissolve the drug with oil phase, using the natural phospholipids as the emulsifier, high-pressure homogenizer and emulsify to produce the emulsion with average particle size less than 600nm. Due to the drug included in the internal oil phase without contact with water and air, it is possible to overcome the shortcomings of insoluble drug with low solubility and poor stability if produced the liquid preparations. Compared with the technology of liposome, submicron emulsion has more advantages of industrialized development; compared with nanoparticle combined with albumin, submicron emulsion with low manufacturing cost, can be sterilized at the terminal and directly used for infusion in clinical treatment without producing precipitation, and is also convenient and safe to use. Therefore, using submicron emulsion as a carrier to develop paclitaxel preparations has bright prospect. Although lots of trials have been carried out by the domestic and overseas researchers and the results have been shown that due to paclitaxel with practical insoluble in water and little solubility in oil (about 0.25mg/ml), if submicron emulsion is prepared directly, the drug loading is less than 0.02 mg/ml, and during the process of sterilization and storage, drug can easily transfer from oil phase to water phase, resulting in demulsification, layered or assembly. Limited by the low solubility of paclitaxel in oil, it is has not been developed so far in the international paclitaxel submicron emulsion with high drug loading, sterilization resistance to heat and pressure as well as stable quality in long-term storage.

In order to improve the solubility of the drug in the oil phase, and breakthrough the limit of physical and chemical properties in paclitaxel submicron emulsion study, we carry out the research on "palitaxel lipid complex" and "paclitaxel submicron emulsion prepared with the lipid complex as the intermediate carrier" in the early stage, and filed two patent applications, "paclitaxel lipid complex" with application number of 200810168213.X and "paclitaxel submicron emulsion prepared with the lipid complex as the intermediate carrier" with application number of 200810168212.5.

With respect to paclitaxel lipid complex as disclosed in Patent application 200810168213.X, the preferred lipid materials is the natural egg yolk lecithin, soybean lecithin and cholesterol, the weight ratio of paclitaxel and lipid materials is 1:1~19, that is amount of lipid materials is 1~19 times higher than that of paclitaxel (using phospholipids as lipid materials, the molar ratio of paclitaxel and lipid materials is 1:2.2~20; using cholesterol as lipid materials, the molar ratio of paclitaxel and lipid materials is 1:2.2~42; using cholic acid as lipid materials, the molar ratio of paclitaxel and lipid materials is 1:2.1~40.) The submicron emulsion as disclosed in Patent application 200810168212.5 is prepared with paclitaxel lipid complex disclosed in Patent application 200810168213.X as the intermediate carrier.

The purpose of preparing paclitaxle lipid complex lies in improving the solubility of paclitaxel in oil, which provides with a good intermediate carrier for the follow-up preparation of submicron emulsion. But the further studies have been shown that the technical solution as disclosed in Patent application 200810168213.X and 200810168212.5 has the following shortcomings:
1. Phospholipids as lipid materials to producing the complex, provided that it has improved the solubility of drug in oil, maximum to 2mg/ml, the higher amount of phospholipids used can not constantly improve the solubility in oil; as the intermediate carrier to producing submicron emulsion, phospholipids with maximum drug loading of 0.5mg/ml, is unstable if stored for 6 months, which can not meet the clinical treatment.
2. Cholesterol as lipid materials to producing the complex is superior to phospholipids in improving the solubility of drug in oil, however cholesterol belongs to steroid, amount of cholesterol is 1-19 times than that of paclitaxel, which has lots of drawbacks: **(1) excessive intakes:** the intake of cholesterol for healthy adults is about 300mg~500mg (equivalent to the amount of cholesterol in 1~2 egg yolks), and the dose of paclitaxel for clinical use once is about 300mg. Compared with cholesterol and its preparations disclosed in Patent application 200810168213.X, the cholesterol intake is about 300mg~5700mg with maximum amount equivalent to 19 egg yolks, and is significantly high with safety risk; **(2) instability of submicron emulsion in the long-term storage:** using cholesterol as the intermediate carrier to producing submicron emulsion, according to the dose for the clinical treatment and the specific concentration of paclitaxel, the higher amount of lipid materials in the complex, the higher total amount of the complex encapsulated in the oil phase. Limited by the droplets volume and oil-water interface in the internal oil phase, when the total amount of the complex exceeds the contents of oil phase and oil-water interface, it is possible for parts of drug to make free to the external water phase, which leads to the low encapsulation efficiency and instability of submicron emulsion. Through the study of the submicron emulsion as disclosed in Patent application 200810168212.5, its encapsulation efficiency is less than 85%, and the quality of submicron emulsion is stable if stored at 4 for 6 months, but the apparent layered has been shown if stored for 12 months, which indicates that the labelled amount has been decreased and the amount of impurity in paclitaxel has been increased; **(3) high cost of preparation:** the high amount of lipid materials and solvent during the process as well as the long time to remove the solvent lead to high cost of manufacture, which does not comply with the principles of pharmacoeconomics.

Therefore, on the basis of the two Patent applications above, the application carries out lots of test research, selects and optimizes the lipid materials and its amount further, in order to provide the complex with fewer quantities of lipid materials and lower cost, which has improved the safety, effectiveness and quality control in clinical practice. The inventors of the present invention have found that in a large number of lipid materials, steroid is the best lipid materials, characterized in that steroid lipid materials has strong capacity to complex with paciltaxel and using fewer quantities of steroid can increase the solubility of drug in oil to the maximum. Using steroid complex described in present invention as the intermediate carrier to producing oil-in-water submicron emulsion, because of the fewer total amounts of the complex encapsulated, higher encapsulation efficiency as well as not easily demulsifciation in the long-term storage, its physical and chemical stability are superior to those of submicron emulsion as disclosed in Patent application 200810168212.5. Said paclitaxel steroid complex in the present invention has laid a solid foundation for the quality control and effectiveness as well as safety in clinical practice of the follow-up submicron emulsion.

### Contents of the Invention

An object of the present invention is to provide with one kind of paclitaxel /steroid complex composed of the paclitaxel and steroid lipid materials. The molar ratio of paclitaxel and steroid stands 1:0.2~4, preferably 1:0.25~2, more preferably 1:0.33~1.

Paclitaxel/steroid complex said the present invention, the steroid lipid materials thereof is selected from the group consisting of the natural steroidal substance or one kind of its derivatives; said natural steroid is selected from the group consisting of cholesterol, 7 - dehydrocholesterol (also known as 7 - hydrogenated cholesterol), lanosterol, sitosterol, stigmasterol, sitosterolum, ergosterol, brassicasterol, mycosterol or oysters steroid; and said its derivative is selected from the group consisting of cholic acid, deoxycholic acid or anthropodesoxycholic acid. The preferred steroid is selected from the group consisting of cholesterol, 7- dehydrogenation cholesterol or ergosterol, the more preferred steroid is selected from the group consisting of cholesterol or ergosterol.

Said paclitaxel/ steroid complex in the present invention may contains antioxidant stabilizer. The preferred antioxidant stabilizer is selected from the group consisting of sodium bisulfite, sodium metabisulfite, vitamin C, EDTA and its salts, vitamin E or its derivatives.

Another object of the present invention lies in providing with the preparation of paclitaxel/ steroid complex, which can be prepared in accordance with the following Method 1 or 2.
Method 1 includes the following steps:
   a. Mix paclitaxel and steroid in proportion, dissolve with an appropriate amount of organic solvent, and then add the antioxidant stabilizer randomly;
   b. Stir under the suitable temperature condition, remove the organic solvent, vacuum drying.
Method 2 includes the following steps:
   a. Dissolve paclitaxel and steroid in an appropriate amount of different organic solvents, mix, add the antioxidant stabilizer randomly;
   b. Stir under the suitable temperature condition, remove the organic solvent by rotary evaporation or spray drying, vacuum drying.

As described in the preparation of the present invention, the organic solvents should be chosen from one king or several of dichloromethane, ethanol, methanol, benzyl alcohol, acetone, ethyl acetate, tetrahydrofuran and tert-butanol; preferably one kind or several of ethanol, acetone, ethyl acetate and tetrahydrofuran. If several of organic solvents used, it refers to a mixture of organic solvents.

In the preparation of the invention, the removal of organic solvents can be obtained by rotary evaporation or spray drying.

In the preparation of the present invention, "mix in proportion" refers to mix paclitaxel and steroid as mentioned above in the present application, that is the molar ratio of paclitaxel and steroid stands 1:0.2~4, preferably 1:0.25~2, more preferably 1:0.33~1; "an appropriate amount" in "an appropriate amount of organic solvents" refers to the amount of mixture for dissolving paclitaxel and steroid determined by those skilled in the art according to the conventional techniques. For example, the concentration of paclitaxel steroid complex in the solution is 0.5~16mg/ml, calculated as paclitaxel, preferably 1.0 ~ 8.0mg/ml; "the suitable temperature condition" refers to 25 -70 , preferably 35-55 , such as 25 ,35 , 45 , 55 or 70 .

In the preparation of the present invention, the stirring and vacuum drying time are subject to those skilled in the art in accordance with the conventional technique, for instance stirring time can be 0.5-3.0 hours, e.g. 0.5 hour, 1.0 hour, 1.5 hours or 2.0 hours, and vacuum drying time can be 8-48 hours, e.g. 8 hours, 12 hours, 16 hours or 24 hours.

In the preparation of the present invention, an appropriate amount of antioxidant stabilizer may be considered to add, and its dose complies with the commonly used in the preparation of liposome complex in the field, generally not more than 1% of the sum of paclitaxel and cholesterol (weight).

The present invention also provides with the use of the paclitaxel/steroid complex for the preparation of oil-in-water submicron emulsion, dry emulsion, self-microemulsifying system or oral preparations. Wherein oil-in-water submicron emulsion or dry emulsion is obtained by dissolving paclitaxel/ steroid complex in the oil phase, which can be administered by injection intended for cancer treatment. The preparation has the advantages of high drug loading, good stability as well as no Cremopher EL in formualtion, and its safety is better than the commercial injection. Self-microemulsifying system can be obtained by dissolving paclitaxel/steroid complex in the oil phase, then adding an appropriate amount of surfactants (emulsifier) and cosurfactant (assisting emulsifying agent) used for cancer treatment through injection, mucosal or oral administration. Oral preparations, in particular solid dosage forms such as capsules or tablets, should be obtained by adding excipients for pharmaceutical use in paclitaxel steroid complex, intended for cancer therapy through oral administration, which has higher bioavailability.

The present invention also provides with the use of the paclitaxel/steroid complex in the present invention for the preparation of anti-cancer drugs, said cancer is selected from the group consisting of ovarian cancer ,breast cancer, non-small cell lung cancer, head and neck cancer, and also for gastric or pancreatic cancer.

Unless specified stated, the meanings of the science and terminology and titles as mentioned in the present invention comply with the general understanding those skilled in the filed; and unless specified pointed out, the substance to be used and its content or proportion, device, instrument, preparation conditions, etc. are well known by those skilled in the art or described in the present invention.

**Said Paclitaxel/ steroid complex in the present invention has the following advantages in particular:**
**1) Less lipid materials used and low preparation cost:** the fully complex of drug and lipid materials is a prerequisite for improvement of drug solubility in oil. Compared with paclitaxel complex known in this field, including paclitaxel complex as diaclosed in Patent application 200810168213.X, the molar ratio of drug and steroid lipid materials stands 1: 0.2~4, preferably 1:0.25~2, more preferably 1:0.33~1.
   The molecular weight of said steroid lipid materials in the present invention is 384.6~414.7, thus the molar ratio of drug and steroid is 1: 0.2, the corresponding weight ratio is 1: 0.09~1: 0.097; whereas the molar ratio is 1:4, the corresponding weight ratio is 1:1.80~1.94, that is the molar ratio of drug and steroid stands 1:0.2~4 in the preferred range, the corresponding weight ratio is 1:0.09~1.94. Accordingly the molar ratio of drug and steroid stands 1:0.25~2 in the preferred range, the corresponding weight ratio is 1: 0.11~0.97; whereas the molar ratio of drug and steroid stands more preferably 1:0.33~1, the corresponding weight ratio is 1:0.15~0.49.
   Compared with the contents as disclosed in Patent application 200810168213.X, the steroid used in the present invention is lipid materials, the weight ratio of drug and lipid materials reduces from 1:1~19 to 1: 0.09~1.94 (preferably 1:0.11~0.97), which decreases the amount of lipid materials, narrows the usage amount, improves the loading of paclitaxel in the complex (increased from 5%~50% to 35%~91.7%), and ensures paclitaxel to be fully complex so that the maximum solubility of paclitaxel can be obtained in oil, which can meet the follow-up preparation of submicron emulsion. Increasing the amount of lipid materials can not continually improve the solubility of the drug in oil.
**2) Less lipid materials intake:** take 300mg of paclitaxel once as calculated, steroid intake should be controlled at 27mg~580mg once, preferably 33mg~290mg. Compared with 300mg~5700mg as disclosed in Patent application 200810168213.X, steroid intake has obviously decreased which can reduce the high risk of safety.
**3) Improving the encapsulation efficiency and stability of submicron emulsion:** The maximum amount of steroid in the complex of the present invention is only 1.94 times the amount of paclitaxel, preferably 0.97 times. If dissolving the compound with vegetable oil to preparing oil-in-water submicron emulsion, the less total quantities of compound within the oil phase can improve the encapsulation efficiency and physical and chemical stability in long-term storage. The comparative research has been proved that using the said complex in the present invention as the intermediate carrier to preparing submicron emulsion, the encapsulation efficiency can be kept at 90% above and the quality stability can be achieved at 4 ° C for 12 months, whereas the encapsulation efficiency of the submicron emulsion as disclosed in Patent application 200810168212.5 kept at 65% -85% and the apparent layered had been shown if stored at 4 ° C for 12 months and the quantity of impurity had been increased greatly.
**4) Enchancing the safety:** Compared with the commercially available injection, the formualtion of submicron emulsion prepared with paclitaxel/ steroid complex as the intermediate carrier excludes Cremopher EL which can avoid a severe allergic reaction triggered by Cremopher EL, reducing animal toxicity and increasing the tolerated dose.

Although the present invention has been fully described in connection with the description of figures and embodiments, it is to be noted that the invention included but not limited to these embodiments as well as the preparation method will become apparent to those skilled in the art. Moreover, according to the description, technicians in the field can equally substitute, combine, change or modify the compound as being included within the scope of the present invention.

### Description of figures

Figure 1: DSC curve of the complex with different molar ratio and the physical mixture in Test Example 3
Figure 2: X -ray diffraction diagram in Test Example 6
Figure 3: Infrared spectrum in Test Example 7
Figure 4:
   Figure 4-1: Ultraviolet spectrum in Test Example 8
   Figure 4-2: Comparison of HPLC chromatographic peaks in Test Example 8 (peak 1: paclitaxel)

### Examples

### Preparation of examples

### Example 1 Paclitaxel cholesterol complex

Take 9.0g of paclitaxel and 0.81g of cholesterol in a rotary evaporator, dissolve with 3000ml of acetone, mix at 40 °Cfor 1 hour, then remove the solvent by rotary evaporation method, vacuum drying at 40 °C for 12 hours.

### Example 2 Paclitaxel cholesterol complex

Take 9.0g of paclitaxel and 0.99g of cholesterol in a rotary evaporator, dissolve with 3000ml of tetrahydrofuran, mix at 25 °C for 1 hour, then remove the solvent by rotary evaporation method, vacuum drying at 25 °C for 12 hours.

### Example 3 Paclitaxel cholesterol complex

Take 8.5g of paclitaxel and 1.275g of cholesterol in a triangle flask, dissolve with 3000ml of tetrahydrofuran, mix at 45°C for 1.5 hours, then remove the solvent by rotary evaporation method, vacuum drying at 45 °C for 16 hours.

### Example 4 Paclitaxel cholesterol complex

Take 8.0g of paclitaxel and 1.84g of cholesterol in a triangle flask, dissolve with 3000ml of acetone, mix at 35°C for 1 hour, then remove the solvent by rotary evaporation method, vacuum drying at 35 °C for 10 hours.

### Example 5 Paclitaxel cholesterol complex

Take 7.0g of paclitaxel and 3.15g of cholesterol in a triangle flask, dissolve with 32500ml of acetone, mix at 50°C for 2 hours, then remove the solvent by rotary evaporation method, vacuum drying at 50 °C for 15 hours.

### Example 6 Paclitaxel cholesterol complex

Take 7.0g of paclitaxel and 4.2g of cholesterol in a triangle flask, dissolve with 5000ml of ethyl acetate, mix at 55°C for 1 hour, then remove the solvent by rotary evaporation method, vacuum drying at 40 °C for 15 hours.

### Example 7 Paclitaxel cholesterol complex

Take 5.2g of paclitaxel and 4.73g of cholesterol in a rotary evaporator, dissolve with 3000ml of dichloromethane, mix at 60°C for 2.5 hours, then remove the solvent by rotary evaporation method, vacuum drying at 55°C for 15 hours.

### Example 8 Paclitaxel cholesterol complex

Take 5.2g of paclitaxel, 4.85g of cholesterol and 0.05g of vitamin E in a rotary evaporator, dissolve with 4000ml of tetrahydrofuran, mix at 40°C for 1 hour, then remove the solvent by spray drying for 15 hours, vacuum drying at 55°C for 15 hours.

### Example 9 Paclitaxel cholesterol complex

Take 5.1g of paclitaxel and 4.95g of cholesterol in a rotary evaporator, dissolve with 5200ml of a mixture of ethanol and tert-butanol, mix at 45°C for 1 hour, then remove the solvent by rotary evaporation method, vacuum drying at 65°C for 15 hours.

### Example 10 Paclitaxel cholesterol complex

Take 5.0g of paclitaxel and 7.51g of cholesterol in a rotary evaporator, dissolve with 5200ml of a mixture of ethanol and tert-butanol" mix at 45°C for 1 hour, then remove the solvent by rotary evaporation method, vacuum drying at 65°C for 15 hours.

### Example 11 Paclitaxel cholesterol complex

Take 5.0g of paclitaxel and 9.10g of cholesterol in a rotary evaporator, dissolve with 5200ml of a mixture of ethanol and tert-butanol" mix at 45°C for 1 hour, then remove the solvent by rotary evaporation method, vacuum drying at 65°C for 15 hours.

### Example 12 Paclitaxel/ 7-dehydrocholesterol complex

Take 5g of paclitaxel, add 0.55g of 7-dehydrocholesterol in a rotary evaporator, dissolve with 500ml of acetone, mix at 45°C for 1 hour, then remove the solvent by rotary evaporation method, vacuum drying at 65°C for 15 hours.

### Example 13 Paclitaxel/ 7-dehydrocholesterol complex

Take 5g of paclitaxel, add 4.85g of 7-dehydrocholesterol in a rotary evaporator, dissolve with 500ml of acetone, mix at 45°C for 1 hour, then remove the solvent by rotary evaporation method, vacuum drying at 65°C for 15 hours.

### Example 14 Paclitaxel/ 7-dehydrocholesterol complex

Take 5g of paclitaxel, add 9.70g of 7-dehydrocholesterol in a rotary evaporator, dissolve with 500ml of acetone, mix at 45°C for 1 hour, then remove the solvent by rotary evaporation method, vacuum drying at 65°C for 15 hours.

### Example 15 Paclitaxel/ ergosterol complex

Take 5g of paclitaxel and 0.54g of ergosterol in a rotary evaporator together, dissolve with 500ml of acetone, mix at 45°C for 1 hour, then remove the solvent by rotary evaporation method, vacuum drying at 65°C for 15 hours.

### Example 16 Paclitaxel/ ergosterol complex

Take 5g of paclitaxel and 4.83g of ergosterol in a rotary evaporator, dissolve with 500ml of acetone, mix at 45°C for 1 hour, then remove the solvent by rotary evaporation method, vacuum drying at 65°C for 15 hours.

### Example 17 Paclitaxel/ ergosterol complex

Take 5g of paclitaxel and 9.10g of ergosterol in a rotary evaporator, dissolve with 500ml of acetone, mix at 45°C for 1 hour, then remove the solvent by rotary evaporation method, vacuum drying at 65°C for 15 hours.

### Example 18 Paclitaxel/ cholic acid complex

Take 4g of paclitaxel each in 5 portions, add 0.5g, 2.0g, 3.0g, 4.6g and 7.70g of cholic acid separately in a rotary evaporator, dissolve with 1000ml of acetone each, mix at 45°C for 1 hour, then remove the solvent by rotary evaporation method, vacuum drying at 65°C for 15 hours.

### Example 19 Submicron emulsion prepared with paclitaxel/ steroid complex as the intermediate carrier

Dissolve 25g of glycerol with approximate 720ml of water for injection, heat to 40-60°C, add 15g of refined soybean lecithin and 30g of poloxamer (188) to a blender, obtain the homogeneous water phase, keep at its temperature; take a quantity of paclitaxel/ steroid complex from Example 1 to 18 (equivalent to 500mg of paclitaxel), add 200ml of soybean oil, heat to 40-60°C, obtain the oil phase. Mix the water phase slowly into the oil phase, stir to producing uniform colostrum. Transfer the colostrum to a homogenizer several times, adjusting pH to 4.0-6.0 with 0.1mol/L of hydrochloric acid, collect all emulsion, sterilize at 115°C for 30 mins, obtain submicron emulsion with 0.5mg/ml of drug loading. The average particle size is 125nm~150nm detected by Lazer particle size analyzer.

### Example 20 Submicron emulsion prepared with paclitaxel/ steroid complex as the intermediate carrier

Dissolve 12.5g of glycerol with approximate 300ml of water for injection, heat to 50°C, add 7.5g of refined egg yolk lecithin and 15g ofpoloxamer (188) to a blender, obtain homogenous water phase, keep at its temperature; take a quantity of paclitaxel/ steroid complex from Example 5, 13 and 16 (equivalent to 500mg of paclitaxel), add 125ml of a mixture containing soybean oil and MEDIUM CHAIN OIL by volume, heat to 50°C, obtain the oil phase. Mix the water phase slowly into the oil phase, stir at high speed to producing uniform colostrum. Transfer the colostrum to a homogenizer several times, adjusting pH to 4.0-6.0 with 0.1mol/L of hydrochloric acid, add water to 500ml, collect all emulsion, sterilize at 115°C for 30 mins, obtain submicron emulsion with 1.0mg/ml of drug loading. The average particle size is 230nm~250nm detected by Lazer particle size analyzer.

### Example 21 Submicron emulsion prepared with paclitaxel/ steroid complex as the intermediate carrier

Dissolve 12.5g of glycerol with approximate 300ml of water for injection, heat to 55°C, add 7.5g of refined soybean lecithin and 15g of poloxamer (188) to a blender, obtain homogenous water phase, keep at its temperature; take a quantity of paclitaxel/ steroid complex from Example 1 to 18 (equivalent to 600mg of paclitaxel), add 125ml of soybean oil, heat to 55°C, obtain the oil phase. Mix the water phase slowly into the oil phase, stir at high speed to producing uniform colostrum. Transfer the colostrum to a homogenizer several times, adjusting pH to 4.0-6.0 with 0.1mol/L hydrochloric acid, add water to 500ml, collect all emulsion, sterilize at 115°C for 30 mins, obtain submicron emulsion with 1.2mg/ml of drug loading. The average particle size is 125nm~133nm detected by Lazer particle size analyzer.

### Example 22 Submicron emulsion prepared with paclitaxel/ steroid complex as the intermediate carrier

Dissolve 12.5g of glycerol with approximate 300ml of water for injection, heat to 60°C, add 7.5g of refined soybean lecithin and 15g ofpoloxamer (188) to a blender, stir at high speed to producing homogenous water phase, keep at its temperature; take a quantity of paclitaxel/ steroid complex from Example 1 to 18 (equivalent to 1000mg of paclitaxel) respectively, add 150ml of soybean oil, heat to 60°C, obtain the oil phase. Mix the water phase slowly into the oil phase, stir at high speed to producing uniform colostrum. Transfer the colostrum quickly to a homogenizer several times, adjusting pH to 4.0-6.0 with 0.1mol/L of hydrochloric acid, add water to 500ml, collect all emulsion, sterilize at 115°C for 30 mins, obtain submicron emulsion with 2.0mg/ml of drug loading. The average particle size is 128nm~141nm detected by Lazer particle size analyzer.

### Example 23 Dry emulsion prepared with paclitaxel/ steroid complex as the intermediate carrier

Take a quantity of non-sterilized submicron emulsion from Example 20 to 22, dissolve with 3% (W/V) of mannitol, sterilze and filter through 0.22µm of microporous membrane, freeze to drying.

### Example 24 Self-microemulsion prepared with paclitaxel/ steroid complex as the intermediate carrier

Take a quantity of paclitaxel/ steroid complex (equivalent to 100mg of paclitaxel) form Example 1-18 respectively, add 5ml of MEDIUM CHAIN OIL, mix to dissolve, add 1.6ml of PEG400 and 0.5ml of Tween-80, mix well, obtain the uniform and transparent self-microemulsifying system.

Take 10ml of self-microemulsifying system as mentioned above, add 5% of glucose injection to 50ml, and then obtain mciroemulsion promptly with particle size less than 100nm.

### Example 25 Capsules prepared with paclitaxel/ steroid complex as the intermediate carrier

Take a quantity of paclitaxel/ steroid complex (equivalent to 250mg of paclitaxel) form Example 1-18 respectively into No.2 hard capsules.

### Example 26 Tablets prepared with paclitaxel/ steroid complex as the intermediate carrier

Take a quantity of paclitaxel/ steroid complex (equivalent to 500mg of paclitaxel) form Example 1-18 respectively, add 500mg of microcrystalline cellulose, 500mg of lactose, and an amount of magnesium stearate, mix well, adjust the punch to compressing 100-250mg of tablets in specification.

### Test Examples

### Test Example 1:

### Technical solution validation as disclosed in China Patent application 200810168213.X

Carry out the preferred lipid materials and its amount as disclosed in *China Patent application* 200810168213.X*,* use soya bean lecithin and cholesterol as lipid materials and weight ratio of drug and lipid materials stands 1:1.85~18.5, prepare the reference complex , investigate the solubility in soybean oil (calculated as paclitaxel), compared with uncombined free paclitaxel.

**Table 1 Validation results from the complex as disclosed in Patent application 200810168213.X**

| Complex | Amount ratio of paclitaxel/ lipid materials | | Solubility in soybean oil (mg/ml) |
|---|---|---|---|
| | Molar ratio | Weight ratio | |
| Paclitaxel/ phospholipids | 1:2 | 1:1.85 | 0.68 |
| | 1:4 | 1:3.70 | 0.75 |
| | 1:6 | 1:5.55 | 2.12 |
| | 1:10 | 1:9.23 | 2.32 |
| | 1:20 | 1:18.46 | 2.25 |
| Paclitaxel/ Cholesterol | 1:4 | 1:1.82 | 9.04 |
| | 1:6 | 1:2.73 | 8.87 |
| | 1:10 | 1:4.53 | 9.15 |
| | 1:20 | 1:9.06 | 8.75 |
| | 1:40 | 1:18.12 | 8.69 |
| Paclitaxel Reference | / | / | 0.27 |

The results has been shown that the amount of phospholipids is 5.55 times that of paclitaxel, solubility intends to be stable and controlled at 2.12 ~ 2.32mg/ml; while the amount of cholesterol is 1.82~18.12 times the weight of paclitaxel, the solubility is controlled at 8.75 ~ 9.15mg/ml, it is suggested that using cholesterol as lipid materials is superior to phospholipids in the aspect of improving the solubility of drug substance in oil and the amount of cholesterol is expected to be further decreased and optimized.

### Test Example 2

### Influencing factors analysis of paclitaxel/ steroid complex in oil

### Amount ratio of paclitaxel and steroid

Take 4g of paclitaxel, add cholesterol, 7-dehydrocholesterol and ergosterol respectively in accordance with the amount ratio as stated in the table 2 below, move to a rotary evaporator together, dissovle with 500ml of acetone each, mix at 45°C for 1 hour then remove the solvent by the rotary evaporation method, vacuum drying at 65°C for 15 hours. Take a quantity of complex, add 20g of soybean oil for injection used, heat in water bath for 1 hour and shake, obtain the saturated solution. Filter the solution, take a quantity of the successive filtrate, add absolute ethanol to volume, inject 20µl into the liquid chromatography, using Kromasil-C18 (250mm×4.6mm,5µm) as the column, acetonitrile-water (54:46) as the mobile phase, flow rate at 1.0ml/min and detection wavelength is 230nm, calculate the solubility ofpaclitaxel in vegetable oil, see results in the table below.

**Table 2 Effect of amount ratio of paclitaxel and steroid on the solubility of the complex in oil**

| Steroid lipid materialss | Amount ratio of paclitaxel and steroid | | Solubility in soybean oil (mg/ml) |
|---|---|---|---|
| | Molar ratio | Weight ratio | |
| Cholesterol | 5:1 ( 1:0.2 ) | 1:0.091 | 7.66 |
| | 4:1 ( 1:0.25 ) | 1:0.112 | 8.25 |
| | 3:1 ( 1:0.33 ) | 1:0.151 | 8.56 |
| | 2:1 ( 1:0.5 ) | 1:0.225 | 9.14 |
| | 1:1 | 1:0.453 | 9.49 |
| | 1:2 | 1:0.906 | 9.06 |
| | 1:4 | 1:1.813 | 9.04 |
| 7-dehydrocholesterol | 5:1 ( 1:0.2 ) | 1:0.090 | 7.47 |
| | 4:1 ( 1:0.25 ) | 1:0.113 | 7.93 |
| | 3:1 ( 1:0.33 ) | 1:0.150 | 8.77 |
| | 2:1 ( 1:0.5 ) | 1:0.225 | 9.21 |
| | 1:1 | 1:0.450 | 8.85 |
| | 1:2 | 1:0.901 | 8.69 |
| | 1:4 | 1:1.802 | 9.12 |
| Ergosterol | 5:1 ( 1:0.2 ) | 1:0.093 | 7.23 |
| | 4:1 ( 1:0.25 ) | 1:0.116 | 8.50 |
| | 3:1 ( 1:0.33 ) | 1:0.155 | 9.26 |
| | 2:1 ( 1:0.5 ) | 1:0.232 | 8.78 |
| | 1:1 | 1:0.465 | 8.59 |
| | 1:2 | 1:0.929 | 9.11 |
| | 1:4 | 1:1.858 | 8.73 |
| Paclitaxel reference | / | / | 0.27 |

The results have been shown that molar ratio of paclitaxel and steroid stands 1: 0.2-4, that is the maximum amount of steroid less than 4 times the molar of paclitaxel, the maximum solubility of drug in oil is obtained.

### Paclitaxel concentration

Using acetone as the solvent, amount molar ratio of paclitaxel and cholesterol is 1:1, the reaction temperature at 35°C, the reaction time is 0.5 h, it is to investigated the effect of paclitaxel concentration of 16.0, 8.0, 4.0, 2.0, 1.0 and 0.5mg/ml on the complex preparation. The results have been shown the concentration of paclitaxel has no obvious effect on the solubility of complex in oil, see the table below.

**Table 3 Effect of paclitaxel concentration on solubility of cholesterol complex in oil**

| Paclitaxel concentration (mg/mL) | Solubility in soybean oil (mg/ml) |
|---|---|
| 16.0 | 8.89 |
| 8.0 | 9.21 |
| 4.0 | 9.58 |
| 2.0 | 9.47 |
| 1.0 | 9.39 |
| 0.5 | 8.44 |

### Test Example 3 Paclitaxel cholesterol complex in DSC curves

Test samples:
- Paclitaxel
- Cholesterol
- Paclitaxel cholesterol complex (complex as prepared in table 2 of Test Example 2 with the molar ratio of 4:1 , 3 :1 , 2:1 , 1:1 , 1:2 , 1:4) ;
- Physical mixture of paclitaxel and cholesterol (take a quatlity of paclitaxel, and cholesterol with the same molar ratio, mix well, intended for comparative research)

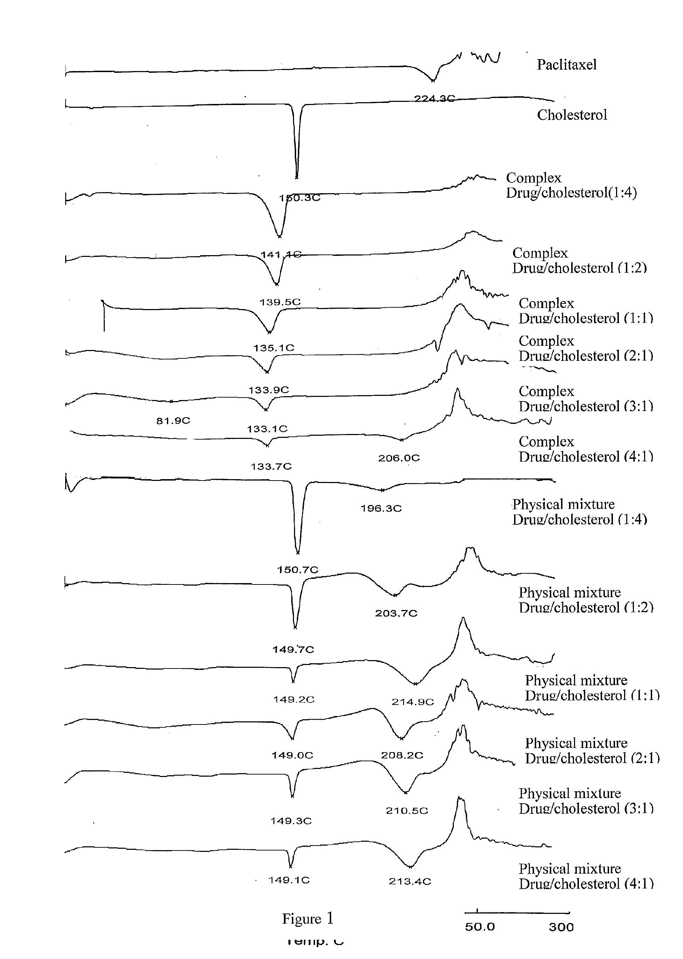

Method: carry out the differential scanning calorimetry (DSC) to detremine the characteristics of DSC curves, temperature at 25~300°C, heating rate is 10°C/min, nitrogen flow rate is 60ml/min; DSC curves have been shown in Figure 1.

Result: endothermic melting peak of paclitaxel is at 225.7°C and that of cholesterol is at 150.9°C. In the preparation of physical mixture in different proportions, the endothermic characteristics of cholesterol has not been changed with the melting point at 149~150°C; whereas the endothermic peak of paclitaxel has a little shift but melting feature remains, it is just a simple physical mixed. The shift reason of melting peak obtained with paclitaxel in the physical mixture as following, the melting point of cholesterol is lower than that of paclitaxel, after cholesterol melting, it has affected the dispersed state of drug, which will change the melting characteristicsof paclitaxel.

In the preparation of complex in different proportions, the melting characteristics of cholesterol has shift obviously, and the endothermic peak of paclitaxel has disappeared completely, which indicates that both have fully complex.

### Test Example 4 Solubility research on paclitaxel cholesterol complex in different oil phases

Take a quantity of paclitaxel cholesterol complex as described in table 2 of Test Example 2, add a mixture of soybean oil for injection, MEDIUM CHAIN OIL and soybean oil/MEDIUM CHAIN OIL (1:1), heat to 60°C, mix well to dissolve, heat in the water bath at 60°C for 1 hour, shaking constantly during the process, obtain the saturated solution. Filter the solution, take a quantity of the successive filtrate, dilute with absolute ethanol, carry out the HPLC method, calculate the solubility of complex in oil; take a quantity of paclitaxel, repeat the operation.. Compared the solubility of complex and paclitaxel, the results have been proved that the solubility in oil can be improved when the drug combining with cholesterol in different proportions, see the table below:

**Table 4 Results of drug solubility in vegetable oil (mg/ml)**

| Drug/Cholesterol | 5 : 1 | 4 : 1 | 3 : 1 | 2 : 1 | 1 : 1 | 1 : 2 | 1 : 4 | Paclitaxel |
|---|---|---|---|---|---|---|---|---|
| Soybean oil | 7.66 | 8.25 | 8.56 | 9.14 | 9.49 | 9.06 | 9.04 | 0.27 |
| MCO | 25.25 | 26.21 | 26.47 | 26.45 | 23.54 | 27.69 | 27.69 | 2.37 |
| Mixed oil* | 15.51 | 13.87 | 14.02 | 16.56 | 15.50 | 16.89 | 16.89 | 1.36 |

| | | | | | | | | |
|---|---|---|---|---|---|---|---|---|
| Note : mixed oil* is a mixture of soybean oil and MEDIUM CHAIN OIL (volume ratio of 1:1) | | | | | | | | |

### Test Example 5 Solubility of paclitaxel cholesterol complex in n-octanol

Take a quantity of paclitaxel cholesterol described in table 2 into 50ml of conical flask, add 10g of n-octanol, shake at 25°C in the thermostatic oscillator for 24 hours, obtain the saturated solution. Take 5ml of the solution, filter by 0.45µm of membrane, take an amount of the successive filtrate, dilute with absolute ethanol, carry out the ultraviolet spectrophotometry method, calculate the apparent solubility of physical mixture and cholesterol in n-octanol, compared the results with paclitaxel. The results have been proved that the solubility in n-octanol can be improved when the drug combining with cholesterol in different proportions, see the table below:

**Table 5 Results of drug solubility in n-octanol (mg/ml, 25°C)**

| Drug/Cholesterol | 4 : 1 | 3:1 1 | 2 : 1 | 1 : 1 | 1 : 2 | 1 : 4 | Paclitaxel |
|---|---|---|---|---|---|---|---|
| Solubility | 23.59 | 24.28 | 20.70 | 25.83 | 28.32 | 32.41 | 8.86 |

### Test Example 6 X-ray diffraction (XRD) analysis of paclitaxel cholesterol complex

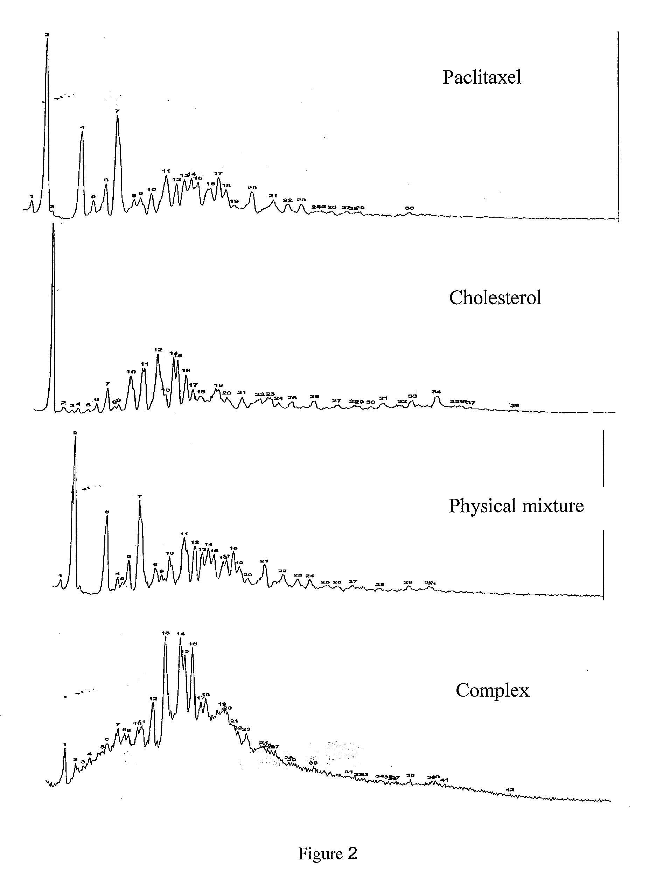

Test samples: paclitaxel, cholesterol, paclitaxel cholesterol complex (sample from Example 5), physical mixture of paclitaxel and cholesterol (molar ratio of 1:1).
Detection condition: Cu-K target, 40kV of tube voltage, 200MA of tube flow, diffraction range is 3°<2θ<60°.
Results: diffraction results are shown in Figure 2.

The results have been clearly stated that characteristic diffraction peaks of paclitaxel in No. 2, 4, 7, diffraction angle (2θ) at 5.480, 8.840 and 12.180, peak intensify is 23567, 11319, 13277; the strongest diffraction peak of cholesterol in No.1, diffraction angle (2θ) at 5.160, peak intensify is 35506; both characteristic diffraction peaks have been obviously shown in the diffraction spectrum, that is the sum profile of paclitaxel and cholesterol profile; but the diffraction spectrum of complex has been changed. The characteristic diffraction peaks intensify of paclitaxel and cholesterol have greatly weaken or peaks have disappeared, new characteristic diffraction peaks have been shown at angle of 15.240, 16.759, 17.160 and 17.960, diffraction peak intensity is 4492, 3588, 2604 and 3186, lower than that of paclitaxel and cholesterol separately. It is indicated that paclitaxel is dispersed at microcrystalline or amorphous state in the complex.

### Test Example 7 Infrared spectrometry (IR) analysis of paclitaxel cholesterol complex

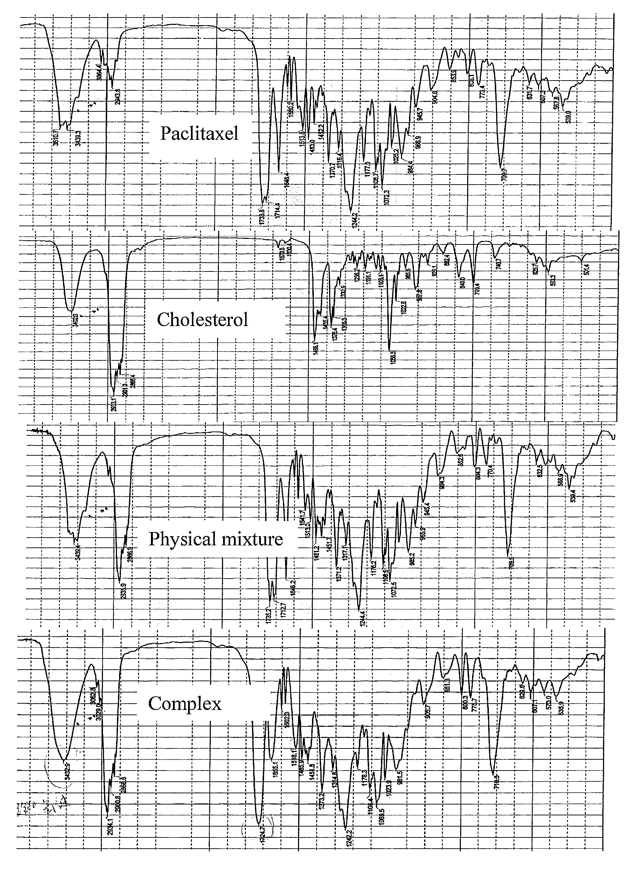

Take paclitaxel, cholesterol, paclitaxel cholesterol complex respectively (sample with molar ratio of 1:1 from Example 5), physical mixture of paclitaxel and cholesterol (molar ratio of 1:1), using potassium bromide to compress, carry out infrared spectroscopy method in the range of 400-4000 cm-1, the results have been shown in Figure 3.

IR spectrum has shown that the main absorption peak of paclitaxel is carbonyl group C = 0 which has two splitting peaks at 1733.8 cm-1 and 1714.4 cm-1, acylamino group has the carbonyl peak at 1646.4 cm-1 as well as stretching vibration absorption peak of oxhydryl group O-H at 3300-3500 cm-1; in chloesterol IR sperum, the characteristic absorption peaks are the staurated C-H bonding strenching vibration peaks at 2933.1cm-1, 2901.0cm-1 and 2866.4cm-1 and strenching vibration absorption peak of oxhydryl group at 3402.3cm-1; the IR specrum of the physical mixture is essentially the sum of absorption peaks obtained with cholesterol and paclitaxel; the charcteristic absorption peaks of the complex has been changed, that is the characteristic peaks of carbonyl group and acylamino group obtained in paclitaxel have been changed, two splitting peaks of carbonyl group convert into a blunt peak with strong absorption at 17247cm-1, the peak shape of carbonyl group in acylamino group has been blunted at 1646.4 cm-1; for cholesterol, absorption peak shape of oxhydryl group has been widened at 3432.9 cm-1 in cholesterol and absorption intensify has been enhanced. It is indicated that carbonyl group of paclitaxel has reacted with oxhydryl group of cholesterol which could be produced a new complex.

### Test exsample 8 UV absorption spectroscopy (UV) and HPLC chromatographic peaks study of cholesterol complex

Dissolve paclitaxel and paclitaxel cholesterol complex (sample with the molar ratio of 1:1 from Example 5) separately with absolute ethanol, using absolute ethanol as blank, carry out UV scanning method in the range of 200~400nm, the results have been shown in Figure 4-1. It is concluded that the UV characteristics of paclitaxel complies with that of paclitaxel cholesterol complex which indicates the chromophore group of paclitaxel in the complex has not been changed, that is two components convert into compound by the intermolecular forces reaction without forming new chemical bonds.

Inject 20µl of ethanol solution of paclitaxel and paclitaxel cholesterol complex described above into the liquid chromatography, using Kromasil-C18 (250mm × 4.6mm, 5µm) as the column, acetonitrile - water (54:46) as the mobile phase, flow rate at 1.0ml / min, detection wavelength is 230nm, record the retention time, results have been shown in Figure 4-2. It is concluded that the retention time of the main peak obtained with paclitaxel complies with that of paclitaxel cholesterol complex, which indicates the free paclitaxel can be ionized from the complex in protonated ethanol solution.

### Test Example 9 Stability study of paclitaxel cholesterol complex

Take solid powder of drug steroid complex prepared from Example 1-18, store at 25°C, sampling periodically and examine the appearance changes. Add absolute ethanol into the solution with suitable concentration, inject 20µl into HPLC, using Kromasil C-18 (250mmx4.6mm, 5µm) as the column, acetonitrile-water (54:46) as the mobile phase, flow rate at 1.0ml/min, detection wavelength is 230nm, determine the content and impurities. The results indicate that there is no significant changes in appearace, content and impurity and it has stable quality compared to the intitial.

**Table 6 Stability of paclitaxel steroid complex in long-term storage**

| Temperature/Time | | Appearance | Content(%) | Degradated products (%) |
|---|---|---|---|---|
| 25°C | 0 month | White powder | 98.05~102.10 | ND |
| | 1 month | White powder | 98.25~102.23 | <0.2 |
| | 3 months | White powder | 97.78~101.74 | <0.3 |
| | 6 months | White powder | 98.90~102.36 | <0.3 |
| | 9 months | White powder | 98.55~101.76 | <0.2 |
| | 12 months | White powder | 98.36~101.87 | <0.2 |
| | 24 months | White powder | 98.71~101.88 | <0.2 |

### Test Example 10

### Test Example 10-1 Paclitaxel steroid complex

Experimental complex 1-6: according to the technical requirements of the present invention, use cholesterol, 7-dehydrocholesterol and ergosterol to preparing the paclitaxel steroid complex with molar ratio of 1:1-1:4. The detailed procedure is as following, take paclitaxel and steroid into a triangle flask, dissolve with 2000ml of acetone, mix at 40°C for 1 hour, remove the solvent by rotary evaporation method, vacuum drying at 40°C for 24 hours.

Reference complex 1-4: according to the technical requirements of Patent application 200810168212.5, use soybean lecithin and cholesterol as lipid materials to preparing 4 groups of reference complex by the same method used for comparative study, wherein the molar ratio of paclitaxel phospholipids complex is 1:6 and 1:10, while the molar ratio of paclitaxel cholesterol complex is 1:10 and 1:20. As detailed in the table below.

**Table 7 Composition of paclitaxel cholesterol complex**

| Complex No./lipid materials | Amount of paclitaxel | Amount of lipid materials | Paclitaxel/ lipid materials | |
|---|---|---|---|---|
| | | | Molar ratio | Weight ratio |
| Experimental complex 1/ cholesterol | 8.0 g | 3.6 g | 1:1 | 1:0.45 |
| Experimental complex 2/ cholesterol | 8.0 g | 7.2 g | 1:2 | 1:0.90 |
| Experimental complex 3/ 7-dehydrocholesterol | 3.0 g | 1.35g | 1:1 | 1:0.45 |
| Experimental complex 4/ 7-dehydrocholesterol | 3.0 g | 5.406g | 1:4 | 1:1.80 |
| Experimental complex 5/ ergosterol | 3.0 g | 1.40g | 1:1 | 1:0.46 |
| Experimental complex 6/ ergosterol | 3.0 g | 5.58g | 1:4 | 1:1.86 |
| Reference complex 1/ phospholipids | 3.0 g | 16.65g | 1:6 | 1:5.55 |
| Reference complex 2/ phospholipids | 3.0 g | 6.78g | 1:10 | 1:9.23 |
| Reference complex 3/ cholesterol | 3.0 g | 13.5g | 1:10 | 1:4.50 |
| Reference complex 4/ cholesterol | 3.0 g | 27.18g | 1:20 | 1:9.06 |

### Test Example 10-2 Paclitaxel submicron emulsion prepared with paclitaxel cholesterol complex as the intermediate carrier

### [Formualtion]

| Ingredients | submicron emulsion 1 | submicron emulsion 2 | submicron emulsion 3 | submicron emulsion 4 |
|---|---|---|---|---|
| Experimental complex 1 * | 145mg | 290mg | 580mg | 1160mg |
| Egg yolk lecithin | 2g | 2.4g | 3g | 3g |
| Poloxamer(188) | 1g | 2g | 4g | 6g |
| Glycerol | 5g | 5g | 5g | 5g |
| Soybean oil | 40ml | 40ml | 50ml | 50ml |
| Add water for injection to | 200ml | 200ml | 200ml | 200ml |
| Total | 200ml | 200ml | 200ml | 200ml |

| | | | | |
|---|---|---|---|---|
| *Experimental complex 1 is the complex with paclitaxel/ cholesterol weight ratio of 1:0.45 prepared from Example 1. | | | | |

### [Preparation]

► take 130-140ml of water for injection, add egg yolk lecithin, Poloxamer (188) and glycerol according to formualtion, transfer to a blender to disperse, obtain homogeneous aqueous phase, heat to 40°C and keep the temperature;
► take a quantity of soybean oil according to formualtion, preheat to 40°C, take paclitaxel cholesterol complex 1 prepared from Example 1, dissolve with preheated soybean oil, transfer to a blender to disperse, obtain homogeneous oil phase;
► add the aqueous phase slowly into the oil phase with constantly stirring, mix at 10,000 rpm / min for 5mins, obtain a homogeneous colostrum, transfer quickly into high-pressure homogenizer, homogenizer 6 times, collect all of emulsion, adjusting pH with 0.1mol/L of hydrochloric acid to 4.0 ± 0.5, add water to 200ml, shake and pack, sterilize at 115°C for 30 minutes,.

For submicron emulsion 1-4, the amount of emulsifier (egg yolk lecithin) is 1.0% (g/ml), 1.2% (g/ml), 1.5% (g/ml) and 1.5% (g/ml) of total amount of submicron emulsion, the amount of cosurfactant Poloxamer (188) is 0.5% (g/ml), 1.0% (g/ml), 2.0% (g/ml) and 3.0% (g/ml) of total amount of submicron emulsion, and the drug loading of paclitaxel is 0.5mg/ml, 1.0mg/ml, 2.0mg/ml, 4.0mg/ml. Determined by Laser particle size analyzer, the average particle size of 4 groups emulsion is 225nm, 233 nm, 245nm and 230nm.

### Test Example 10-3 Paclitaxel submicron emulsion prepared with paclitaxel cholesterol complex as the intermediate carrier

### [Formualtion]

| Ingredients | submicron emulsion 5 | submicron emulsion 6 | submicron emulsion 7 | submicron emulsion 8 |
|---|---|---|---|---|
| Experimental | 190mg | 380mg | 760mg | 1520mg |
| complex 2* Egg yolk lecithin | 2g | 2.4g | 3g | 3g |
| Poloxamer(188) | 2.4g | 4g | 4g | 6g |
| Glycerol | 5g | 5g | 5g | 5g |
| Vitamin E | / | / | / | 40mg |
| Soybean oil | 40ml | 40ml | 50ml | 50ml |
| Add water for injection to | 200ml | 200ml | 200ml | 200ml |
| Total | 200ml | 200ml | 200ml | 200ml |

| | | | | |
|---|---|---|---|---|
| *Experimental complex 2 is the complex with paclitaxel cholesterol complex weight ratio of 1:0.90 prepared from Example 1. | | | | |

### [Preparation]

► take 130-140ml of water for injection, add Poloxamer (188) and glycerol according to formualtion, transfer to a blender to disperse, obtain homogeneous aqueous phase, heat to 80°C and keep the temperature;
► take a quantity of soybean oil according to formualtion, preheat to 80°C, take complex 2, egg yolk lecithin and vitamin E according to formulation, dissolve with preheated soybean oil, transfer to a blender to disperse, obtain homogeneous oil phase;
► add the aqueous phase slowly into the oil phase with constantly stirring, 20,000 rpm / min for 5mins, obtain a homogeneous colostrum, transfer quickly into high-pressure homogenizer, homogenizer 6 times, collect all of emulsion, adjusting pH with 0.1mol/L of hydrochloric acid to 5.5 ± 0.5, add water to 200ml, shake and pack, sterilize at 115°C for 30 minutes.

For submicron emulsion 5-8, the amount of emulsifier (egg yolk lecithin) is 1.0% (g/ml), 1.2% (g/ml), 1.5% (g/ml) and 1.5% (g/ml) of total amount of submicron emulsion, the amount of cosurfactant Poloxamer (188) is 1.2% (g/ml), 2.0% (g/ml), 2.0% (g/ml) and 3.0% (g/ml) of total amount of submicron emulsion, and the drug loading of paclitaxel is 0.5mg/ml, 1.0mg/ml, 2.0mg/ml, 4.0mg/ml. Determined by Laser particle size analyzer, the average particle size of 4 groups emulsion is 246nm, 262 nm, 231nm, 242nm.

### Test Example 10-4 Paclitaxel submicron emulsion prepared with paclitaxel cholesterol complex as the intermediate carrier

### [Formualtion]

| Ingredients | submicron emulsion 9 | submicron emulsion 10 | submicron emulsion 11 | submicron emulsion 12 |
|---|---|---|---|---|
| Experimental complex 1* | 145mg | 290mg | 580mg | 1450mg |
| Poloxamer(188) | 4g | 4g | 4g | 4g |
| Glycerol | 5g | 5g | 5g | 5g |
| Vitamin E | / | / | / | 40mg |
| MCO | 40ml | 40ml | 50ml | 50ml |
| Add water for injection to | 200ml | 200ml | 200ml | 200ml |
| Total | 200ml | 200ml | 200ml | 200ml |

| | | | | |
|---|---|---|---|---|
| *Experimental complex 1 is the complex with paclitaxel cholesterol weight ratio of 1:0.45 prepared from Example 1. MCO : medium chain oil | | | | |

### [Preparation]

The same procedure as Test Example 10-3, wherein adjusting pH to 5.0 ± 0.5.

For submicron emulsion 9-12, the amount of emulsifier (soybean lecithin) is 1.2% (g/ml), 1.2% (g/ml), 1.2% (g/ml) and 1.5% (g/ml) of total amount of submicron emulsion, the amount of cosurfactant Poloxamer (188) is 2.0% (g/ml) of total amount of submicron emulsion, and the drug loading of paclitaxel is 0.5mg/ml, 1.0mg/ml, 2.0mg/ml and 5.0mg/ml. Determined by Laser particle size analyzer, the average particle size of 4 groups emulsion is 165nm, 153nm, 127nm, 138nm.

### Test Example 10-5 Paclitaxel submicron emulsion prepared with paclitaxel cholesterol complex as the intermediate carrier

### [Formualtion]

| Ingredients | submicron emulsion 13 | submicron emulsion 14 | submicron emulsion 15 | submicron emulsion 16 |
|---|---|---|---|---|
| Experimental complex 1* | 290mg | 435mg | 580mg | 1450mg |
| soybean lecithin | 2.4g | 2.4g | 2.4g | 4.0g |
| Poloxamer(188) | 3g | 3g | 4g | 4g |
| Glycerol | 5g | 5g | 5g | 5g |
| Oil mixture** | 40ml | 40ml | 40ml | 50ml |
| Add water for injection to | 200ml | 200ml | 200ml | 200ml |
| Total | 200ml | 200ml | 200ml | 200ml |

| | | | | |
|---|---|---|---|---|
| *Experimental complex 1 is the complex with paclitaxel cholesterol weight ratio of 1:0.45 prepared from Example 1. **Oil mixture is a mixture of soybean oil and medium chain oil (volume ratio of 1:1). | | | | |

### [Preparation]

The same procedure as Example 3, wherein adjusting pH to 4.5 ± 0.5.

For submicron emulsion 13-16, the amount of emulsifier (soybean lecithin) is 1.2% (g/ml), 1.2% (g/ml), 1.2% (g/ml) and 2.0% (g/ml) of total amount of submicron emulsion, the amount of cosurfactant Poloxamer (188) is 1.5%(g/ml), 1.5%(g/ml), 2.0%(g/ml) and 2.0% (g/ml) of total amount of submicron emulsion, and the drug loading of paclitaxel is 1.0mg/ml, 1.5mg/ml, 2.0mg/ml and 5.0mg/ml. Determined by Laser particle size analyzer, the average particle size of 4 groups emulsion is 145nm, 138nm, 133nm, 146nm.

### Test Example 10-6 Paclitaxel submicron emulsion prepared with paclitaxel cholesterol complex as the intermediate carrier

### [Formualtion]

| Ingredients | submicron emulsion 17 | submicron emulsion 18 | submicron emulsion 19 | submicron emulsion 20 |
|---|---|---|---|---|
| Experimental complex 2* | 190mg | 380mg | 760mg | 1520mg |
| egg yolk lecithin | 3.0g | 3.0g | 4.0g | 6.0g |
| Poloxamer(188) | 4g | 4g | 6g | 6g |
| Glycerol | 5g | 5g | 5g | 5g |
| Oil mixture** | 30ml | 40ml | 50ml | 60ml |
| Add water for injection to | 200ml | 200ml | 200ml | 200ml |
| Total | 200ml | 200ml | 200ml | 200ml |

| | | | | |
|---|---|---|---|---|
| *Experimental complex 2 is the complex with paclitaxel cholesterol weight ratio of 1:0.90 prepared from Example 1. **Oil mixture is a mixture of soybean oil and MEDIUM CHAIN OIL (volume ratio of 1:1). | | | | |

### [Preparation]

The same procedure as Example 2, wherein adjusting pH to 5.5 ± 0.5.

For submicron emulsion 17-20, the amount of emulsifier (egg yolk lecithin) is 1.5% (g/ml), 1.5% (g/ml), 2.0% (g/ml) and 3.0% (g/ml) of total amount of submicron emulsion, the amount of cosurfactant Poloxamer (188) is 2.0%(g/ml), 2.0%(g/ml), 3.0%(g/ml) and 3.0% (g/ml) of total amount of submicron emulsion, and the drug loading of paclitaxel is 0.5mg/ml, 1.0mg/ml, 2.0mg/ml and 5.0mg/ml. Determined by Laser particle size analyzer, the average particle size of 4 groups emulsion is 255nm, 263nm, 285nm, 232nm.

### Test Example 10-7 Paclitaxel submicron emulsion prepared with paclitaxel cholesterol complex as the intermediate carrier

### [Formualtion]

| Ingredients | submicron emulsion 21 | submicron emulsion 22 | submicron emulsion 23 | submicron emulsion 24 |
|---|---|---|---|---|
| Experimental complex 1* | 145mg | 290mg | 580mg | 1160mg |
| Fatty glyceride | 3.0g | 4.0g | / | / |
| Polyoxyethylene sorbitan fatty acid ester | / | / | 4.0g | 6.0g |
| Poloxamer(188) | 3g | 4g | 4g | 6g |
| Glycerol | 5g | 5g | 5g | 5g |
| Oleic acid | 0.2g | 0.2g | 0.2g | 0.2g |
| Oil mixture** | 30ml | 40ml | 50ml | 60ml |
| Add water for injection to | 200ml | 200ml | 200ml | 200ml |
| Total | 200ml | 200ml | 200ml | 200ml |

| | | | | |
|---|---|---|---|---|
| *Experimental complex 1 is the complex with paclitaxel cholesterol weight ratio of 1:0.45 prepared from Example 1. **Oil mixture is a mixture of soybean oil and medium chain oil (volume ratio of 1:1). | | | | |

### [Preparation]

### The same procedure as Example 3

For submicron emulsion 21 and 22, the amount of emulsifier (fatty glyceride) is 1.5% (g/ml) and 2.0% (g/ml) of total amount of submicron emulsion; for submicron emulsion 23 and 24, the amount of emulsifier (polyoxyethylene sorbitan fatty acid ester) is 2.0% (g/ml) and 3.0% (g/ml) of total amount of submicron emulsion; for submicron emulsion 21-24, the amount of cosurfactant Poloxamer (188) is 1.5%(g/ml), 2.0%(g/ml), 2.0%(g/ml) and 3.0% (g/ml) of total amount of submicron emulsion, and the drug loading of paclitaxel is 0.5mg/ml, 1.0mg/ml, 2.0mg/ml and 4.0mg/ml. Determined by Laser particle size analyzer, the average particle size of 4 groups emulsion is 145nm, 133nm, 126nm, 158nm.

### Test Example 10-8 Submicron emulsion prepared with 7-dehydrocholesterol or ergosterol as the intermediate carrier

### [Formualtion]

| Ingredients | submicron emulsion 25 | submicron emulsion 26 | submicron emulsion 27 | submicron emulsion 28 |
|---|---|---|---|---|
| Experimental complex 3* | 290mg | / | / | / |
| Experimental complex 4* | / | 5604mg | / | / |
| Experimental complex 5* | / | / | 292mg | / |
| Experimental complex 6* | / | / | / | 5720mg |
| Soybean lecithin | 2.4g | 2.4g | 2.4g | 4.0g |
| Poloxamer(188) | 3g | 3g | 4g | 4g |
| Glycerol | 5g | 5g | 5g | 5g |
| Oil mixture** | 40ml | 40ml | 40ml | 50ml |
| Add water for injection to | 200ml | 200ml | 200ml | 200ml |
| Total | 200ml | 200ml | 200ml | 200ml |

| | | | | |
|---|---|---|---|---|
| *Experimental complex 3-6 is a mixture of paclitaxel 7-dehydrocholesterol complex and paclitaxel ergosterol complex prepared from Example 1. **Oil mixture is a mixture of soybean oil and MEDIUM CHAIN OIL (volume ratio of 1:1). | | | | |

### [Preparation]

### The same procedure as Example 5

For submicron emulsion 25 and 28, the drug loading of paclitaxel is 1.0mg/ml. Determined by Laser particle size analyzer, the average particle size of 4 groups of emulsion is 143nm, 138nm, 141nm, 132nm.

### Test Example 10-9 Paclitaxel submicron emulsion prepared with the reference complex as the intermediate carrier

Take the reference complex 1-4 prepared from Test Example 1, carry out the method with respect to the preparation of submicron emulsion as described in Example above, obtain submicron emulsion 29-32 with drug loading of 5, 1.0, 1.0 and 2.0mg/ml, used for comparative study.

The results of the detailed formulation, preparation method and encapsulation efficiency have been shown as following:

### [Formualtion]

| Ingredients | submicron emulsion 29 | submicron emulsion 30 | submicron emulsion 31 | submicron emulsion 32 |
|---|---|---|---|---|
| Reference complex 1 * | 655mg | / | / | / |
| Reference complex 2* | / | 1023mg | / | / |
| Reference complex 3* | / | / | 1100mg | / |
| Reference complex 4* | / | / | / | 4024mg |
| Egg yolk lecithin | 3g | 3g | 3g | 3g |
| Poloxamer(188) | 3g | 3g | 3g | 3g |
| Glycerol | 5g | 5g | 5g | 5g |
| Soybean oil | 40ml | 40ml | 50ml | 50ml |
| Add water for injection to | 200ml | 200ml | 200ml | 200ml |
| Total | 200ml | 200ml | 200ml | 200ml |

| | | | | |
|---|---|---|---|---|
| *Reference complex 1 and 2 are two groups of reference complex with paclitaxel/ phospholipids weight ratio of 1:5.55 and 1:9.23 prepared from Test Example 1. *Reference complex 3and 4 are two groups of the reference complexs with cholesterol/ paclitaxel phospholipids weight ratio of 1:4.50 and 1:9.06 prepared from Test Example 1. | | | | |

### [Preparation]

► take 130-140ml of water for injection, add egg yolk lecithin, Poloxamer (188) and glycerol according to formualtion, transfer to a blender to disperse, obtain homogeneous aqueous phase, heat to 40-80°C and keep the temperature;
► take the quantity of soybean oil according to formualtion, preheat to 40-80 °C, take paclitaxel cholesterol complex 1 prepared from Example 1, dissolve with preheated soybean oil, transfer to a blender to disperse, obtain homogeneous oil phase;
► add the aqueous phase slowly into the oil phase with constantly stirring, mix at 10,000-20,000 rpm / min for 5-10mins, obtain homogeneous colostrum, transfer quickly into high-pressure homogenizer, homogenizer 6 times, collect all of emulsion, adjusting pH with 0.1mol/L of hydrochloric acid to 4.5 ± 0.5, add water to 200ml, shake and pack, sterilize at 115°C for 30 minutes.

### The stability research and comparison of submicron emulsion prepared in Test Example s above

Take 28 groups of submicron emulsion from Test Example s 10-2 to 10-8 and 4 groups of submicron emulsion from Test Example 10-9, store at 4°C for 12 months respectively, sampling at 0, 6, 12 months, carry out the method as described below to examine the changes in appearance, particle size, content and impurity.

Characteristics: Visual examination, record oil droplets or layered appeared in the surface complying with the colour of submicron emulsion described.

Particle size: take submicron emulsion, examine the particle size using MASTER SIZER 2000 laser particle size analyzer (malvern).

Content and related substances: Take accurately the quantity of paclitaxel submicron emulsion, add anhydrous ethanol to demulsification, obtain the suitable concentration of the test solution. Inject accurately 20µl of the test solution into chromatography, carry out HPLC method, using Kromasil-C18(300mm×4.6mm, 5µm) as the column, acetonitrile - water (54:46) as the mobile phase, flow rate at 1.0ml/min, detection wavelength is 230nm, the column temperature is the room temperature, record the chromatogram, calculate the content of drug substance in emulsion with respect to the peak by the external standard method, caculate the content of impurity by normalization method.

Results: as detailed in the table below

**Table 8 Comparative results of submicron emulsion stability**

| **Sample No./drug loading** | **Initial ( examine within one week after preparation )** | | | | **Stored at 4°C for 12 months** | | | |
|---|---|---|---|---|---|---|---|---|
| | **Appearance** | **Particle size** | **content** | **impurity** | **Appearance** | **Particle size** | **content** | **impurity** |
| Emulsion 1 /0.5 mg/ml | Uniform emulsion | 225nm | 100.2% | 0.33% | Uniform emulsion | 221nm | 99.7% | 0.67% |
| Emulsion 2 /1.0mg/ml | Uniform emulsion | 233nm | 97.6% | 0.31% | Uniform emulsion | 245nm | 97.3% | 0.62% |
| Emulsion 3 /2.0mg/ml | Uniform emulsion | 245nm | 98.5% | 0.31% | Uniform emulsion | 236nm | 98.2% | 0.64% |
| Emulsion 4 /3.0mg/ml | Uniform emulsion | 230nm | 97.6% | 0.37% | Uniform emulsion | 237nm | 97.0% | 0.93% |
| Emulsion 5 10.5mg/ml | Uniform emulsion | 246nm | 99.5% | 0.35% | Uniform emulsion | 228nm | 98.8% | 0.52% |
| Emulsion 6/1.0mg/ml | Uniform emulsion | 262nm | 100.3% | 0.30% | Uniform emulsion | 255nm | 99.5% | 0.61% |
| Emulsion 7 /2.0mg/ml | Uniform emulsion | 231nm | 98.1% | 0.36% | Uniform emulsion | 240nm | 98.6% | 0.57% |
| Emulsion 8 /4.0mg/ml | Uniform emulsion | 242nm | 99.6% | 0.42% | Uniform emulsion | 251nm | 97.3% | 1.01% |
| Emulsion 9 10.5mg/ml | Uniform emulsion | 165nm | 98.8% | 0.35% | Uniform emulsion | 126nm | 99.2% | 0.56% |
| Emulsion 10 /1.0mg/ml | Uniform emulsion | 153nm | 101.6% | 0.30% | Uniform emulsion | 133nm | 100.7% | 0.52% |
| Emulsion 11 /2.0mg/ml | Uniform emulsion | 127nm | 102.2% | 0.32% | Uniform emulsion | 131nm | 100.9% | 0.55% |
| Emulsion 12 /5.0mg/ml | Uniform emulsion | 138nm | 99.8% | 0.45% | Uniform emulsion | 155nm | 99.4% | 1.24% |
| Emulsion 13 /1.0mg/ml | Uniform emulsion | 145nm | 97.4% | 0.31% | Uniform emulsion | 138nm | 98.0% | 0.57% |
| Emulsion 14 /1.5mg/ml | Uniform emulsion | 138nm | 100.3% | 0.35% | Uniform emulsion | 136nm | 98.7% | 0.65% |
| Emulsion 15 /2.0mg/ml | Uniform emulsion | 133nm | 99.2% | 0.31% | Uniform emulsion | 137nm | 98.5% | 0.63% |
| Emulsion 16 /5.0mg/ml | Uniform emulsion | 146nm | 98.5% | 0.43% | Uniform emulsion | 173nm | 97.2% | 1.68% |
| Emulsion 17 10.5mg/ml | Uniform emulsion | 255nm | 97.8% | 0.32% | Uniform emulsion | 244nm | 97.6% | 0.57% |
| Emulsion 18 /1.0mg/ml | Uniform emulsion | 263nm | 99.2% | 0.36% | Uniform emulsion | 259nm | 99.5% | 0.53% |
| Emulsion 19 /2.0mg/ml | Uniform emulsion | 285nm | 100.4% | 0.33% | Uniform emulsion | 272nm | 98.8% | 0.61% |
| Emulsion 20 /5.0mg/ml | Uniform emulsion | 232nm | 101.4% | 0.48% | Uniform emulsion | 258nm | 97.8% | 1.76% |
| Emulsion 21 /0.5mg/ml | Uniform emulsion | 145nm | 98.7% | 0.36% | Uniform emulsion | 136nm | 99.1% | 0.59% |
| Emulsion 22 /1.0mg/ml | Uniform emulsion | 133nm | 98.2% | 0.32% | Uniform emulsion | 127nm | 97.6% | 0.57% |
| Emulsion 23 /2.0mg/ml | Uniform emulsion | 126nm | 101.1% | 0.38% | Uniform emulsion | 134nm | 99.5% | 0.56% |
| Emulsion 24 /4.0mg/ml | Uniform emulsion | 158nm | 98.8% | 0.41% | Uniform emulsion | 163nm | 97.9% | 1.25% |
| Emulsion 25 /1.0mg/ml | Uniform emulsion | 143nm | 99.5% | 0.33% | Uniform emulsion | 128nm | 98.9% | 0.56% |
| Emulsion 26 /1.0mg/ml | Uniform emulsion | 138nm | 97.8% | 0.35% | Uniform emulsion | 132nm | 97.4% | 0.58% |
| Emulsion 27 /1.0mg/ml | Uniform emulsion | 141nm | 99.4% | 0.30% | Uniform emulsion | 145nm | 99.1% | 0.61% |
| Emulsion 28 /1.0mg/ml | Uniform emulsion | 132nm | 98.6% | 0.36% | Uniform emulsion | 126nm | 98.2% | 0.54% |
| Emulsion 29 /0.5mg/ml | Uniform emulsion | 253nm | 97.5% | 0.92% | Layered with floading oil | 323nm | 90.8% | 7.63% |
| Emulsion 30 /1.0mg/ml | Layered | 522nm | 93.2% | 6.27% | Oil-water separation | Not detected | Not detected | Not detected |
| Emulsion 31 /1.0mg/ml | Uniform emulsion | 247nm | 98.6% | 0.62% | Uniform emulsion | 263nm | 94.7% | 3.58% |
| Emulsion 32 /2.0mg/ml | Uniform emulsion | 266nm | 98.9% | 0.78% | Slightly layered | 311nm | 92.8% | 4.64% |

Submicron emulsion 1-28 prepared with steroidal complex as the intermediate carrier in the present invention, store in the refrigerator (4°C) for 12 months, compared with the initial. 1) For emulsion with 5.0mg/ml of drug loading, it is to be noted that the average particle size intends to be greater. The emulsion has not layered and particle size, appearance and content has not obviously changed, impurity increases but not exceeds 2.0%; 2) emulsion with 4.0mg/ml of drug loading has not layered and no obvious changes in particle size, appearance and content, impurity increases but not exceeds 1.3%; 3) emulsion with 3mg/ml of drug loading has not layered and no obvious changes in particle size, appearance and content, impurity not exceeds 1.0%; 4) emulsion with 2mg/ml or less than 2mg/ml of drug loading has no obvious changes in particle size, appearance and content, impurity less than 0.7%.

Submicron emulsions prepared with reference paclitaxel/ phospholipids complex as carrier, 1) the uniform emulsion (submicron emulsion 29) has been formed if the drug loading of 0.5mg/ml, placed for 6 months, there is no obvious changes in appearance and particle size and impurity increases to 3.0%, whereas placed for 12 months, particle size has significant changed and impurity increases to 7% or above, the content decreases with layered and floating oil; 2) if the drug loading increased into 1.0mg/ml, the uniform emulsion has not formed (submicron emulsion 30), the drug crystallization and oil droplets has been appeared at the beginning.

Submicron emulsions prepared with reference paclitaxel/ cholesterol complex as carrier, 1) the uniform emulsion (sample 31-32) has been formed when drug loading of 1.0mg/ml and 2.0mg/ml, placed for 6 months, there is no obvious changes in appearance, particle size and content has not obviously changed and impurity increases but not exceeds 1.5%; 2) placed for 12 months, particle size has significant changed, content decreases and impurity increases to 3.58% and 4.64%, wherein emulsion with drug loading of 2.0mg/ml has slightly layered.

## Claims

1. A paclitaxel steroid complex, **characterized in that**, the complex is composed of paclitaxel and steroid with molar ratio of 1:0.2~4.

2. The paclitaxel steroid complex according to claim 1, **characterized in that**, the molar ratio of paclitaxel and steroid is 1:0.25~2.

3. The paclitaxel steroid complex according to claim 2, **characterized in that**, the molar ratio of paclitaxel and steroid is 1:0.33~1.

4. The paclitaxel steroid complex according to any one of claims 1-3, **characterized in that**, said steroid is the natural steroids or its derivatives.

5. The paclitaxel steroid complex according to claim 4, **characterized in that**, said the natural steroid is selected from the group consisting of cholesterol, 7- dehydrocholesterol, lanosterol, sitosterol, brassicasterol, mycosterol, oysters steroid, stigmasterol, sitosterolum or ergosterol ; said the natural steroid derivative is selected from the group consisting of cholic acid, deoxycholic acid or anthropodesoxycholic acid.

6. The paclitaxel steroid complex according to any one of claims 1-5, **characterized in that**, said complex contains antioxidant stabilizer.

7. The paclitaxel steroid complex according to claim 6, **characterized in that**, said antioxidant stabilizer is selected from the group consisting of sodium bisulfite, sodium metabisulfite, vitamin C, EDTA and its salts, vitamin E and its derivatives.

8. The preparation of paclitaxel steroid complex according to any one of claims 1-7, **characterized in that**, the preparation process includes the following steps:
a. mix paclitaxel and steroid lipid materials in proportion, add organic solvent to dissolve, add antioxidant stabilizer randomly;
b. stir at the suitable temperature, remove the organic solvent, vacuum drying.

9. The preparation according to claim 8, **characterized in that**, during the step a), take quantity of paclitaxel and steroid, dissolve with different organic solvents and mix.

10. The preparation according to any one of claims 8-9, **characterized in that**, said organic solvent is selected from the group consisting of dichloromethane, ethanol, methanol, benzyl alcohol, acetone, ethyl acetate, tetrahydrofuran or tert-butanol.

11. The preparation according to any one of claims 8-9, **characterized in that**, said organic solvent is selected from the group consisting of ethanol, acetone, ethyl acetate or tetrahydrofuran.

12. Use of the paclitaxel steroid complex according to any one of claims 1-7 for the preparation of oil-in-water submicron emulsion, dry emulsion, self microemulsifying system or oral preparations.
